# EUROPÄISCHE PATENTSCHRIFT

(11) **EP 1 928 458 B1**
(45) Veröffentlichungstag und Bekanntmachung des Hinweises auf die Patenterteilung: **04.03.2009**
(21) Anmeldenummer: 06791935.7
(22) Anmeldetag: 08.09.2006
(51) Int. Cl.: A61K 31/47, A61P 25/28

(54) **VERWENDUNG VON INHIBITOREN DES NA+/H+ AUSTAUSCHERS, SUBTYP 5 (NHE5) ZUR GEDÄCHTNISVERBESSERUNG**
USE OF INHIBITORS OF THE NA+/H+ EXCHANGER, SUBTYPE 5 (NHE5), TO IMPROVE MEMORY RETENTION
UTILISATION D'INHIBITEURS DE L'ECHANGEUR NA+/H+, SOUS-TYPE 5 (NHE5) POUR AMELIORER LA MEMOIRE

(30) Priorität: 20.09.2005 DE 102005044815
(43) Veröffentlichungstag der Anmeldung: 11.06.2008
(73) Patentinhaber: Sanofi-Aventis, 75013 Paris (FR)
(72) Erfinder: KLEEMANN, Heinz-Werner, 65926 Frankfurt am Main (DE); LANG, Hans-Jochen, 65719 Hofheim (DE); HEINELT, Uwe, 65926 Frankfurt am main (DE); HOFMEISTER, Armin, 65926 Frankfurt am main (DE); GAUL, Holger, 65926 Frankfurt am Main (DE); SCHROEDER, Ulrich, Hendrich, 97776 Eussenheim (DE); REYMANN, Klaus, 39167 Niederndodeleben (DE)
(74) Vertreter: Kujath, Eckard
(86) Internationale Anmeldenummer: PCT/EP2006/008771
(87) Internationale Veröffentlichungsnummer: WO 2007/033774

(56) Entgegenhaltungen:
- WO-A-01/32624
- WO-A-01/32625
- WO-A-03/033016
- WO-A-03/055880
- WO-A-20/07033773

## Beschreibung

Die Erfindung betrifft die Verwendung von Inhibitoren des Na⁺/H⁺ Austauschers, Subtyp 5 (NHE5) zur Herstellung eines Medikaments zur Behandlung von neurodegenerativen Erkrankungen, Gedächtnisstörungen und Demenzerkrankungen, sowie zur Gedächtnisverbesserung.

Der Begriff Demenz bezeichnet einen Verfall der geistigen Leistungsfähigkeit. Man versteht darunter vor allem die Abnahme von Gedächtnisleistung und Denkvermögen. Die Altersdemenz oder senile Demenz bezeichnet einen fortschreitenden, erworbenen geistigen Abbau bei Personen höheren Alters, welcher auf strukturelle und/oder metabolische Abnormitäten im Zentralnervensystem zurückzuführen ist. Ungefähr 7% der Bevölkerung über 65 Jahre leidet an einer unterschiedlich ausgeprägten Demenz. Die Ursachen für Demenzen sind unterschiedlich: Die Alzheimer-Krankheit stellt mit bis zu 50% die häufigste Form dar, gefolgt von vaskulären Demenzen, wie Multi-Infarkt-Demenz, und Kombinationen dieser beiden Formen. Viel seltenere Ursachen sind Tau-Mutationen, Prionen-Erkrankungen, Polyglutamin-Expansions-Erkrankungen, wie Chorea Huntington und spinocerebelläre Ataxien, und Parkinson. Weiterhin sind auch sekundäre Demenzen nach und/oder bei Infekten (z.B. mit HIV), Hirntraumen, Hirntumoren oder Intoxikationen (z.B. mit Alkohol) bekannt.

Das Konzept der Gedächtniskonsolidierung beruht darauf, dass neugebildete Gedächtnisinhalte sich über die Zeit stabilisieren können und dadurch weniger empfänglich für Störungen durch neue Informationen und Fehlfunktionen des Gehirns werden. Mit Hilfe des vorherrschenden zellulären Modells der Langzeitpotenzierung (LTP) können wesentliche Aspekte und Mechanismen der Gedächtnisbildung und - konsolidierung untersucht werden (Neuroscientist. 9: 463-474. 2003; Brain Res Brain Res Rev. 45: 30-37, 2004; Physiol Rev. 84: 87-136, 2004).

Eine der wichtigsten Hirnregionen, in denen Informationen gespeichert und verarbeitet werden, ist die Hippocampusformation. Es ist lange bekannt, dass im Hippocampus bestimmte Muster elektrischer Stimulation (Tetanisierung) zu Veränderungen der synaptischen Effizienz führen (Bliss und Lomo, J Physiol. 232: 331-356, 1973), die heute als 'Langzeitpotenzierung' oder 'LTP' bezeichnet werden, und die nachfolgend in anderen. Gehirngebieten verschiedenster Säugetiere sowohl in vitro als auch in vivo bestätigt wurden. Die LTP wird heute als wichtige Komponente des neuronalen Mechanismus angesehen, der dem Lernen und dem Gedächtnis zu Grunde liegt. Weiterhin ist bekannt, dass eine schwache LTP mit dem Kurzzeitgedächtnis, und eine starke LTP mit dem Langzeitgedächtnis korreliert (J Neurosci. 20: 7631-7639, 2000; Proc Natl Acad Sci U S A. 97: 8116-8121, 2000).

Der Hippokampus spielt eine zentrale Rolle bei episodischen, räumlichen und deklarativen Lern- und Gedächtnisvorgängen, er ist essentiell für die räumliche Orientierung und Erinnerung von räumlichen Strukturen und spielt eine wichtige Rolle bei der Kontrolle autonomer und vegetativer Funktionen (McEwen 1999, Stress and hippocampal plasticity, Annual Review of Neuroscience 22: 105-122). Bei Demenzerkrankungen des Menschen sind zumeist die Lern- und Gedächtnisprozesse gestört, an denen der Hippocampus beteiligt ist. Ähnliches wurde auch in Tierexperimenten an anderen Säugern gezeigt.
So konnte gezeigt werden, dass alte Mäuse im Vergleich zu jungen Mäusen Defizite im räumlichen Gedächtnis und in der LTP aufweisen, und dass Substanzen, die die LTP verbesserten, gleichzeitig die Gedächtnisdefizite verminderten (Bach et al. 1999, Age-related defects in spatial memory are correlated with defects in the late phase of hippocampal long-term potentiation in vitro and are attenuated by drugs that enhance the cAMP signaling pathway, Proc Natl Acad Sci U S A. 27; 96: 5280-5; Fujii & Sumikawa 2001, Acute and chronic nicotine exposure reverse age-related declines in the induction of long-term potentiation in the rat hippocampus, Brain Res. 894: 347-53; Clayton et al. 2002, A hippocampal NR2B deficit can mimic age-related changes in long-term potentiation and spatial learning in the Fischer 344 rat, J Neurosci.22: 3628-37).

An transgenen Tieren und durch die Applikation von Beta-Amyloidpeptiden konnte in vivo und in vitro gezeigt werden, dass die Peptide LTP verschlechtern bzw. deren Aufrechterhaltung stören (Ye & Qiao 1999, Suppressive action produced by betaamyloid peptide fragment 31-35 on long-term potentiation in rat hippocampus is N-methyl-D-aspartate receptor-independent: it's offset by (-)huperzine A, Neurosci Lett. 275: 187-90; Rowan et al 2003, Synaptic plasticity in animal models of early Alzheimer's disease, Philos Trans R Soc Lond B Biol Sci. 358: 821-8; Gureviciene et al. 2004, Normal induction but accelerated decay of LTP in APP + PS1 transgenic mice, Neurobiol Dis 15: 188-95). Die Beeinträchtigung der LTP bzw. der Gedächtnifunktionen konnte durch Rolipram und Cholinesteraseinhibitoren, wie sie auch in der humanen AlzheimerTherapie eingesetzt werden, korrigiert werden (Gong et al. 2004, Persistent improvement in synaptic and cognitive functions in an Alzheimer mouse model after rolipram treatment, J Clin Invest. 114:1624-34.)

Es ist somit zu erwarten, dass Substanzen, die die LTP verbessern, auch eine positive Wirkung bei Erkrankungen haben, die mit kognitiven Störungen und Demenz einhergehen.

Überraschend wurde gefunden, dass Inhibitoren des zelluläre NHE5 die LTP verstärken. Damit ist eine gedächtnisverbessernde Wirkung des Inhibitors bei Demenzerkrankungen wie Alzheimer und Alzheimer-ähnlichen Formen der Demenz zu erwarten. Der Einsatz eines NHE5 Inhibitors hat gegenüber den bei diesen Erkrankungen bisher eingesetzten Wirkstoffen, wie Acetylcholinesterasehemmern, den Vorteil, dass systemische Wirkungen voraussichtlich gering sein werden oder fehlen, da der NHE5 nur in Neuronen exprimiert ist und daher gehirnspezifisch ist (Am. J. Physiol. Cell. Physiol. 281: C1146-C1157, 2001).

Daher eignen sich NHE5 Inhibitoren zur Behandlung von neurodegenerativen Krankheiten, Gedächtnisstörungen und Demenzerkrankungen wie Altersdemenz, Alzheimer, vaskulären Demenzen, wie zum Beispiel Multi-Infarkt-Demenz, Kombinationen von Alzheimer und cerebrovaskulären Erkrankungen, Tau-Mutationen, Prionen-Erkrankungen, Polyglutamin-Expansions-Erkrankungen, wie zum Beispiel Chorea Huntington und spinocerebelläre Ataxien, und Parkinson, sowie zur Gedächtnisverbesserung. Weiterhin eignen sich NHE5 Inhibitoren zur Behandlung sekundärer Demenzen nach und/oder bei Infektionen, wie zum Beispiel mit HIV, Hirntraumen, Hirntumoren oder Intoxikationen, wie zum Beispiel mit Alkohol.

Zur Behandlung von neurodegenerativen Erkrankungen, Gedächtnisstörungen und Demenzerkrankungen, sowie zur Gedächtnisverbesserung, sind insbesondere die folgenden NHE5 Inhibitoren der Formel I geeignet 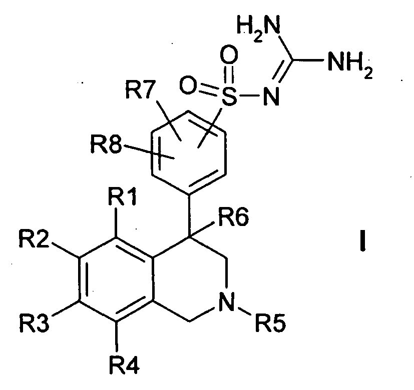 worin bedeuten:
R1, R2, R3 und R4
unabhängig voneinander Wasserstoff, F, Cl, Br, l, CN, NO₂ oder R11-(CₘH₂ₘ)-Aₙ-;
   - m: Null, 1, 2, 3 oder 4;
   - n: Null oder 1;
   - R11: Wasserstoff, Methyl oder CpF₂ₚ₊₁;
   - A: Sauerstoff, NH, N(CH₃) oder S(O)_{q};
   p 1, 2 oder 3;
   q Null, 1 oder 2;
   - R5: Wasserstoff, Alkyl mit 1, 2, 3, 4, 5 oder 6 C-Atomen oder Cycloalkyl mit 3, 4, 5 oder 6 C Atomen;
   - R6: Wasserstoff, OH, F, CF₃, Alkyl mit 1, 2, 3 oder 4 C-Atomen oder Cycloalkyl mit 3, 4, 5 oder 6 C Atomen;
R7 und R8
unabhängig voneinander Wasserstoff, F, Cl, Br, CN, CO₂R12, NR13R14 oder R16-(CₘₘH₂ₘₘ)-Eₙₙ-;
- R12: Wasserstoff, Alkyl mit 1, 2, 3 oder 4 C-Atomen oder Cycloalkyl mit 3, 4, 5 oder 6 C-Atomen;
- R13 und R14: unabhängig voneinander Wasserstoff, Alkyl mit 1, 2, 3 oder 4 C-Atomen oder Cycloalkyl mit 3, 4, 5 oder 6 C-Atomen;
oder
- R13 und R14: bilden mit dem Stickstoffatom, an das sie gebunden sind, einen 4-, 5-, 6- oder 7-gliedrigen Ring, in dem eine CH₂-Gruppe durch NR15, S oder Sauerstoff ersetzt sein kann;
R15 Wasserstoff, Alkyl mit 1, 2, 3 oder 4 C-Atomen oder Cycloalkyl mit 3, 4, 5 oder 6 C-Atomen;
- mm: Null, 1, 2, 3 oder 4;
- nn: Null oder 1;
- R16: Wasserstoff, Methyl oder CₚₚF₂ₚₚ₊₁;
- E: Sauerstoff oder S(O)_{qq};
pp 1, 2 oder 3;
qq Null, 1 oder 2;
sowie deren pharmazeutisch verträgliche Salze und Trifluoracetate.

In einer Ausführungsform sind Verbindungen der Formel I bevorzugt, worin bedeuten
R1, R2, R3 und R4
unabhängig voneinander Wasserstoff, F, Cl, Br, CN oder R11-(CₘH₂ₘ)-Aₙ-;
- m: Null oder 1;
- n: Null oder 1;
- R11: Wasserstoff, Methyl oder CₚF₂ₚ₊₁;
- A: Sauerstoff, NCH₃ oder S(O)_{q};
p 1 oder 2;
q Null, 1 oder 2;
- R5: Wasserstoff, Methyl, Ethyl oder Cyclopropyl;
- R6: Wasserstoff oder Methyl;
- R7 und R8: unabhängig voneinander Wasserstoff, F, Cl, CN, CO₂R12, NR13R14 oder R16-(CₘₘH₂ₘₘ)-Eₙₙ-;
R12 Wasserstoff, Methyl oder Ethyl;
R13 und R14 unabhängig voneinander Wasserstoff, Alkyl mit 1, 2, 3 oder 4 C-Atomen oder Cycloalkyl mit 3, 4, 5 oder 6 C-Atomen; oder
- R13 und R14: bilden mit dem Stickstoffatom, an das sie gebunden sind, einen 5-, 6- oder 7-gliedrigen Ring, in dem eine CH₂-Gruppe durch NR15, S oder Sauerstoff ersetzt sein kann;
R15 Wasserstoff, Alkyl mit 1, 2, 3 oder 4 C-Atomen oder Cycloalkyl mit 3, 4, 5 oder 6 C-Atomen;
- mm: Null, 1 oder 2;
- nn: Null oder 1;
- R16: Wasserstoff, Methyl oder CₚₚF₂ₚₚ₊₁;
- E: Sauerstoff oder S(O)_{qq};
pp 1 oder 2;
qq Null, 1 oder 2; sowie deren pharmazeutisch verträglichen Salze und Trifluoracetate.

Besonders bevorzugt sind Verbindungen der Formel I, worin bedeuten
- R1 und R3: Wasserstoff;
- R2 und R4: unabhängig voneinander Wasserstoff, F, Cl, NH₂, NHCH₃ oder N(CH₃)₂;
- R5: Wasserstoff, Methyl, Ethyl oder Cyclopropyl;;
- R6: Wasserstoff oder Methyl;
- R7 und R8: Wasserstoff;
sowie deren pharmazeutisch verträglichen Salze und Trifluoracetate.

Speziell bevorzugt wird N-Diaminomethylen-4-(6,8-dichloro-2-methyl-1,2,3,4-tetrahydro-isochinolin-4-yl)-benzolsulfonamid sowie dessen pharmazeutisch verträglichen Salze und Trifluoracetate.

In einer Ausführungsform sind Verbindungen der Formel I bevorzugt, in denen die Reste R1, R2, R3 und R4 unabhängig voneinander beschrieben werden durch Wasserstoff, F, Cl, Br, CN oder R11-(CₘH₂ₘ)-Aₙ-, wobei m und n unabhängig voneinander Null oder 1 sind, R11 Wasserstoff, Methyl oder CₚF₂ₚ₊₁ ist und A Sauerstoff, NCH₃ oder S(O)_{q} ist, wobei p 1 oder 2 und q Null, 1 oder 2 sind; besonders bevorzugt sind Verbindungen der Formel I, in denen R1 und R3 Wasserstoff sind und R2 und R4 unabhängig voneinander Wasserstoff, F, Cl, NH₂, NHCH₃ oder N(CH₃)₂, beispielsweise Cl. In einer Ausführungsform sind Verbindungen der Formel bevorzugt, in denen R2 und R4 nicht Wasserstoff sind.

In einer weiteren Ausführungsform sind Verbindungen der Formel 1 bevorzugt, in denen R5 durch Wasserstoff, Methyl, Ethyl oder Cyclopropyl beschrieben wird, beispielsweise Methyl.

In einer weiteren Ausführungsform sind Verbindungen der Formel 1 bevorzugt, in denen R6 durch Wasserstoff oder Methyl beschrieben wird.

In einer weiteren Ausführungsform sind Verbindungen der Formel I bevorzugt, in denen die Reste R7 und R8 unabhängig voneinander beschrieben werden durch Wasserstoff, F, Cl, CN, CO₂R12, NR13R14 oder R16-(CₘₘH₂ₘₘ)-Eₙₙ-, wobei R12 Wasserstoff, Methyl oder Ethyl ist, R13 und R14 unabhängig voneinander Wasserstoff, Alkyl mit 1, 2, 3 oder 4 C-Atomen oder Cycloalkyl mit 3, 4, 5 oder 6 C-Atomen sind oder R13 und R14 zusammen mit dem Stickstoffatom, an das sie gebunden sind, einen 5-, 6- oder 7- gliedrigen Ring bilden, in dem eine CH₂-Gruppe durch NR15, S oder Sauerstoff ersetzt sein kann und wobei R15 Wasserstoff, Alkyl mit 1, 2, 3 oder 4 C-Atomen oder Cycloalkyl mit 3, 4, 5 oder 6 C-Atomen ist, und wobei mm Null, 1 oder 2 ist, nn Null oder 1 ist und R16 Wasserstoff, Methyl oder CₚₚF₂ₚₚ₊₁ ist, wobei E Sauerstoff oder S(O)_{qq} ist, wobei pp 1 oder 2 und qq Null, 1 oder 2 ist; besonders bevorzugt sind Verbindungen der Formel I, in denen R7 und R8 Wasserstoff sind.

Enthalten die Verbindungen der Formel I ein oder mehrere Asymmetriezentren, so können diese unabhängig voneinander sowohl S als auch R konfiguriert sein. Die Verbindungen können als optische Isomere, als Diastereomere, als Racemate oder als Gemische in allen Verhältnissen derselben vorliegen.

Die vorliegende Erfindung umfasst alle möglichen tautomeren Formen der Verbindungen der Formel I.

Die vorliegende Erfindung umfasst weiterhin die Derivate der Verbindungen der Formel I, nämlich Solvate, Hydrate und alle Kristallmodifikationen der Verbindungen der Formel I.

Alkylreste können geradkettig oder verzweigt sein. Dies gilt auch, wenn sie Substituenten tragen oder als Substituenten anderer Reste auftreten, beispielsweise in Fluoralkylresten oder Alkoxyresten. Beispiele für Alkylreste sind Methyl, Ethyl, n-Propyl, Isopropyl (= 1-Methylethyl), n-Buty), Isobutyl (= 2-Methylpropyl), sec-Butyl (= 1-Methylpropyl), tert-Butyl (= 1,1-Dimethylethyl), n-Pentyl, Isopentyl, tert-Pentyl, Neopentyl und Hekyl. Bevorzugte Alkylreste sind Methyl, Ethyl, n-Propyl, Isopropyl und n-Butyl. In Alkylresten können ein oder mehrere, zum Beispiel 1, 2, 3, 4, 5, 6, 7, 8, 9, 10, 11, 12, 13 oder 14, Wasserstoffatome durch Fluoratome substituiert sein. Beispiele für solche Fluoralkylreste sind Trifluormethyl, 2,2,2-Trifluorethyl, Pentafluorethyl, Heptafluorisopropyl. Substituierte Alkylreste können in beliebigen Positionen substituiert sein.

Alkylenreste, wie beispielsweise CₘH₂ₘ, CₘₘH₂ₘₘ oder CᵣH₂ᵣ, können geradkettig oder verzweigt sein. Dies gilt auch, wenn sie Substituenten tragen oder als Substituenten anderer Reste auftreten, beispielsweise in Fluoralkylenresten, wie beispielweise in CₚF₂ₚ und CₚₚF₂ₚₚ. Beispiele für Alkylenreste sind Methylen, Ethylen, 1-Methylmethylen, Propylen, 1-Methylethylen, Butylen, 1-Propylmethylen, 1-Ethyl-1-methylmethylen, 1,2-Dimethylethylen, 1,1-Dimethylmethylen, 1-Ethylethylen, 1-Methylpropylen, 2-Methylpropylen, Pentylen, 1-Butylmethylen, 1-Propylethylen, 1-Methyl-2-ethylethylen, 1,2-Dimethylpropylen, 1,3-Dimethylpropylen, 2,2-Dimethylpropylen, Hexylen und 1-Methylpentylen. In Alkylenresten können ein oder mehrere, zum Beispiel 1, 2, 3, 4, 5, 6, 7, 8, 9, 10, 11 oder 12 Wasserstoffatome durch Fluoratome substituiert sein. Substituierte Alkylenreste können in beliebigen Positionen substituiert sein. In den Alkylenresten können eine oder mehrere CH₂-Gruppen durch Sauerstoff, S, NH, N-Alkyl oder N-Cycloalkyl ersetzt sein.

Beispiele für Cycloalkylreste sind Cyclopropyl, Cyclobutyl, Cyclopentyl oder Cyclohexyl. In Cycloalkylresten können ein oder mehrere, zum Beispiel 1, 2, 3, 4, 5, 6, 7, 8, 9, 10, 11 oder 12 Wasserstoffatome durch Fluoratome substituiert sein. Substituierte Cycloalkylreste können in beliebigen Positionen substituiert sein. Cycloalkylreste können auch verzweigt als Alkyl-cycloalkyl oder Cycloalkyl-alkyl vorliegen, zum Beispiel Methyl-cyclohexyl oder Cyclohexyl-methyl.

Beispiele für Ringe aus NR13R14, wobei R13 und R14 mit dem Stickstoffatom, an das sie gebunden sind, einen 4-, 5-, 6- oder 7-gliedrigen Ring bilden, in dem eine CH₂-Gruppe durch NR15, S oder Sauerstoff ersetzt sein kann, sind Morpholin, Pyrrolidin, Piperidin, Piperazin und N-Methylpiperazin.

Wenn eine Variable mehr als einmal als Komponente vorkommt, sind die Definitionen der Variable bei jedem Auftreten unabhängig voneinander.

Enthalten die Verbindungen der Formel I eine oder mehrere saure oder basische Gruppen bzw. einen oder mehrere basische Heterocyclen, so gehören auch die entsprechenden physiologisch oder toxikologisch verträglichen Salze zur Erfindung, insbesondere die pharmazeutisch verwendbaren Salze. So können die Verbindungen der Formel I an einer sauren Gruppe deprotoniert werden und beispielsweise als Alkalimetallsalze, vorzugsweise Natrium- oder Kaliumsalze, oder als Ammoniumsalze, zum Beispiel als Salze mit Ammoniak oder organischen Aminen oder Aminosäuren, verwendet werden. Da Verbindungen der Formel 1 sets wenigstens eine basische Gruppe enthalten, können sie auch in Form ihrer physiologisch verträglichen Säureadditionssalze z. B. mit folgenden Säuren hergestellt werden: aus anorganischen Säuren wie Salzsäure, Schwefelsäure oder Phosphorsäure oder aus organischen Säuren wie Essigsäure, Zitronensäure, Weinsäure, Milchsäure, Malonsäure, Methansulfonsäure, Fumarsäure. Als Säureadditionssalze kommen dabei Salze aller pharmakologisch verträglichen Säuren in Frage, beispielsweise Halogenide, insbesondere Hydrochloride, Lactate, Sulfate, Citrate, Tartrate, Acetate, Phosphate, Methylsulfonate, p-Toluolsulfonate, Adipinate, Fumarate, Gluconate, Glutamate, Glycerolphosphate, Maleinate und Pamoate (diese Gruppe entspricht auch den physiologisch akzeptablen Anionen); aber auch Trifluoracetate.

Die hier beschriebenen Verbindungen der Formel I lassen sich herstellen durch Chlorsulfonierung von Verbindungen der Formel VIII mittels dem Fachmann bekannter Verfahren mit anschließender Umsetzung mit Guanidin nach dem Fachmann bekannten Verfahren (wie z.B. in Synthetic Communications, 33(7), 1073; 2003 beschrieben).

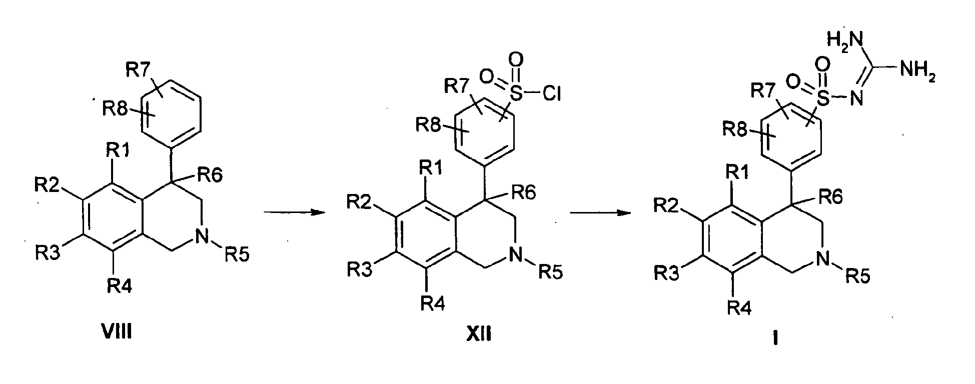

Die nach der Chlorsulfonierung erhaltene Zwischenstufe der Formel XII braucht dabei nicht isoliert zu werden, sondern kann direkt weiter mit Guanidin umgesetzt werden.

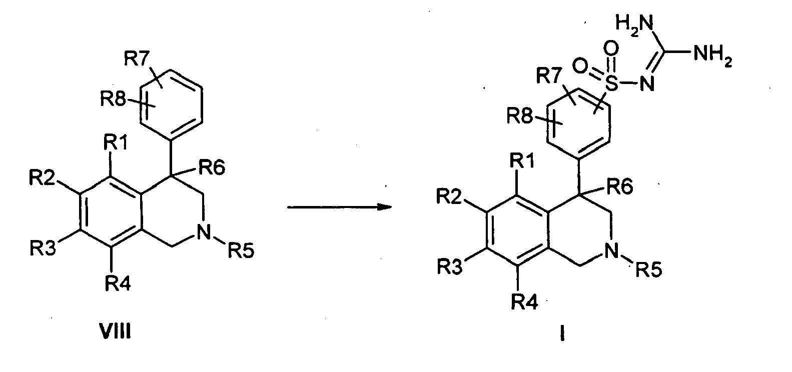

Die Ausgangsverbindungen der Formel VIII können wie folgt hergestellt werden:

Durch Reduktion der Carbonylgruppierung in Verbindungen der Formel VI, zum Beispiel mit Natriumborhydrid, und anschließende säure- oder basenekatalysierte Cyclisierung der entstandenen Alkohole der Formel VII (vgl. Tetrahedron Lett. 1989, 30, 5837; Org. Prep. Proced. Int. 1995, 27, 513) können Tetrahydroisochinoline der Formel VIIIa nach dem Fachmann bekannten Verfahren hergestellt werden, wobei R1 bis R8 die oben angegebene Bedeutung besitzen.

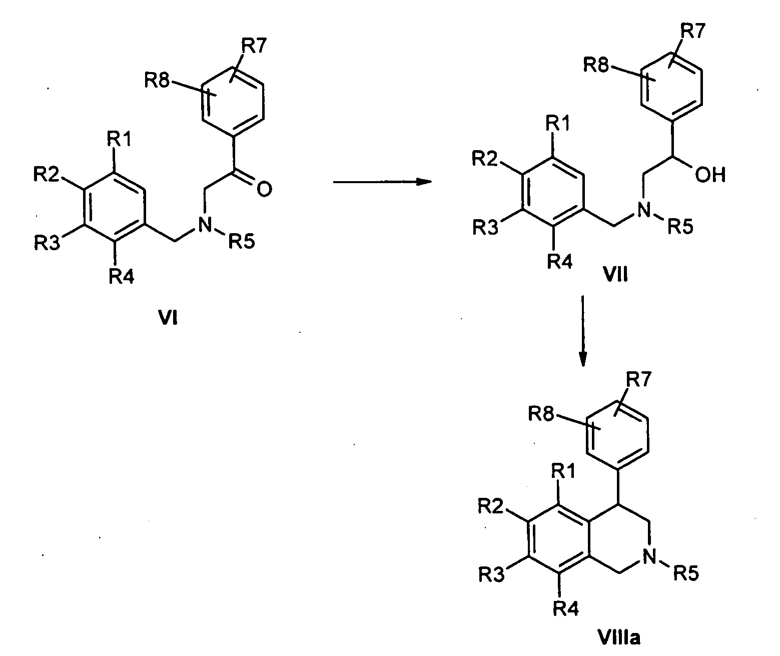

Zur Herstellung alkylverzweigter Verbindungen der Formel I, in denen R6 nicht Wasserstoff ist, können die entsprechenden Diphenylessigsäureester der Formel IX in alpha-Stellung mit R6 nach bekannten Methoden alkyliert werden. Die Verbindungen der Formel X können durch Standardverfahren in die entsprechenden Amide der Formel XI überführt werden, welche in einer Pictet-Spengler-analogen Reaktion in die gewünschten Tetrahydroisochinoline der Formel VIIIb überführt werden (vgl. Tetrahedron 1987, 43, 439; Chem. Pharm. Bull. 1985, 33, 340),
wobei R1 bis R8 wie oben definiert sind und LG einer bei Alkylierungen gängigen Fluchtgruppe, wie beispielsweise Bromid, Chlorid, Tosylat oder Mesylat, entspricht.

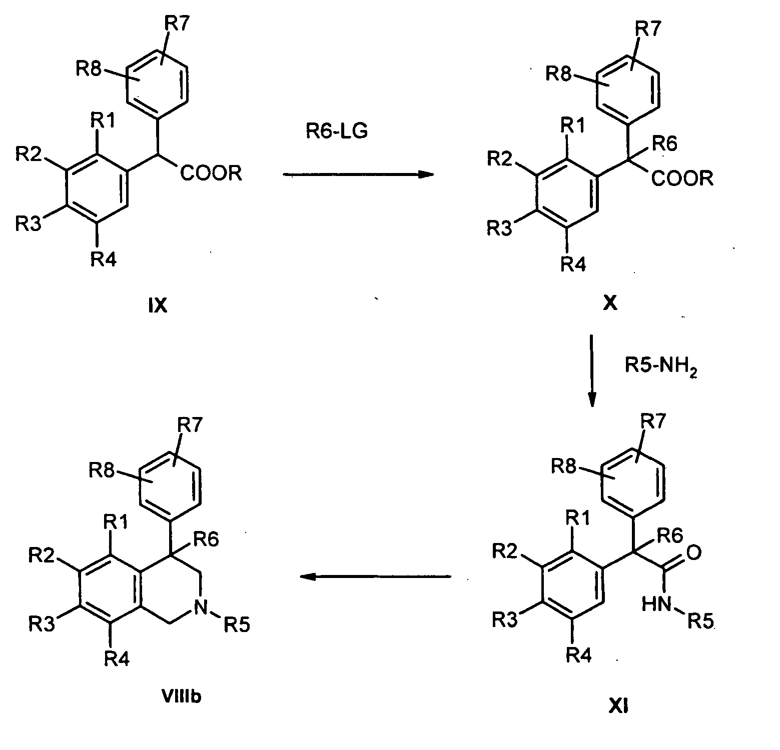

Die oben eingesetzten Verbindungen der Formel VI werden vorzugsweise aus Benzylaminen der Formel IV in dem Fachmann bekannter Weise und den entsprechenden Amino-substituierten alpha-Bromacetophenon-Verbindungen der Formel V hergestellt,
wobei R1 bis R8 wie oben definiert sind,

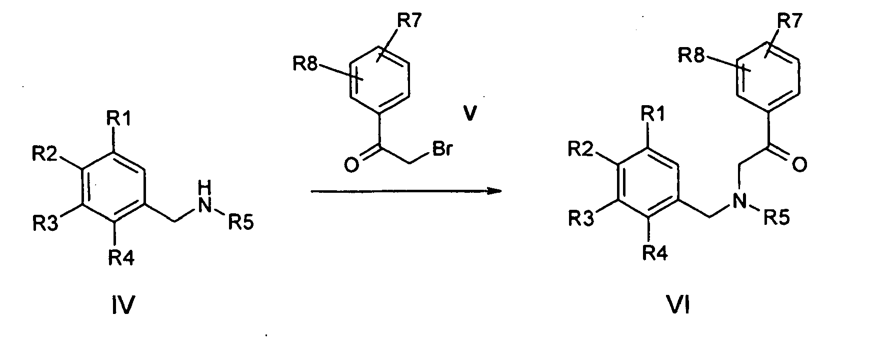

Die alpha-Bromacetophenon-Verbindungen der Formel V lassen sich in literaturbekannten Verfahren aus den entsprechenden Acetophenon-Vorläufern durch Bromierung gewinnen.

Die Benzylamin-Vorläufer der Formel IV können, falls nicht käuflich erhältlich, nach dem Fachmann bekannten Standardverfahren aus den entsprechenden Benzylhalogeniden, beispielweise Benzylchloriden oder -bromiden, der Formel III und den entsprechenden Aminen R5-NH₂ synthetisiert werden,
wobei R1 bis R5 wie oben definiert sind und X F, Cl, Br oder 1, insbesondere Cl oder Br ist.

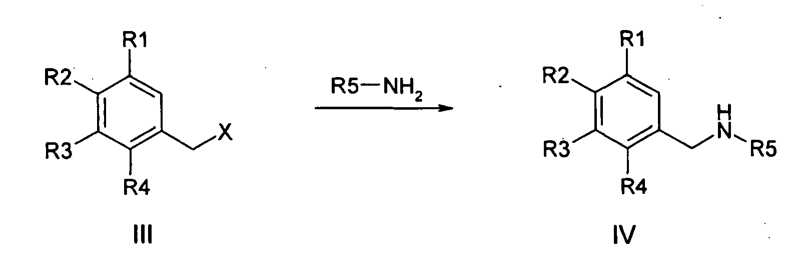

Alternativ sind Verbindungen der Formel IV auch durch reduktive Aminierung eines Aldehyds der Formel IIIa nach dem Fachmann bekannten Standardverfahren zugänglich,
wobei R1 bis R5 wie oben definiert sind.

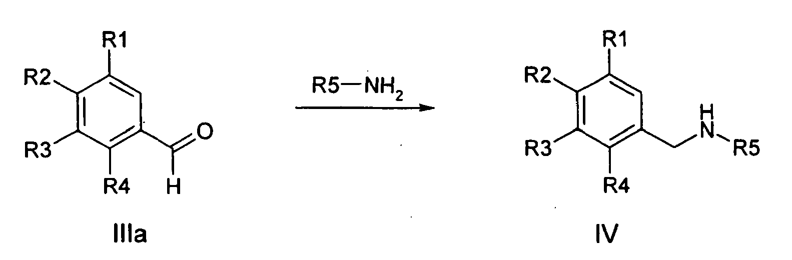

Die Verbindungen der Formeln III und IIIa, IX und R6-LG. und R5-NH₂ sind käuflich erhältlich oder können nach oder analog zu in der Literatur beschriebenen, dem Fachmann bekannten Verfahren hergestellt werden.

Die Aufarbeitung und gewünschtenfalls die Reinigung der Produkte und/oder Zwischenprodukte erfolgt nach den üblichen Methoden wie Extraktion, Chromatographie oder Kristallisation und den üblichen Trocknungen.

Die Erfindung betrifft die Verwendung der Verbindungen der Formel I und deren pharmazeutisch verträgliche Salze zur Herstellung eines Medikaments zur Behandlung von neurodegenerativen Krankheiten, Gedächtnisstörungen und Demenzerkrankungen wie Altersdemenz, Alzheimer, vaskulären Demenzen, wie zum Beispiel Multi-Infarkt-Demenz, Kombinationen von Alzheimer und cereborvaskulären Erkrankungen, Tau-Mutationen, Prionen-Erkrankungen, Polyglutamin-Expansions-Erkrankungen, wie zum Beispiel Chorea Huntington und spinocerebelläre Ataxien, und Parkinson, sowie zur Gedächtnisverbesserung. Weiterhin eignen sich diese NHE5 Inhibitoren zur Behandlung sekundärer Demenzen nach und/oder bei Infektionen, wie zum Beispiel mit HIV, Hirntraumen, Hirntumoren oder Intoxikationen, wie zum Beispiel mit Alkohol.

Die Erfindung betrifft auch Heilmittel für die humane oder veterinäre Anwendung gemäss der Ansprüchen enthaltend eine wirksame Menge einer Verbindung der Formel 1 und / oder eines pharmazeutisch verträglichen Salzes davon, ebenso wie Heilmittel für die humane oder veterinäre Anwendung gemäss der Ansprüchen enthaltend eine wirksame Menge einer Verbindung der Formel I und / oder eines pharmazeutisch verträglichen Salzes davon allein oder in Kombination mit einem oder mehreren anderen pharmakologischen Wirkstoffen oder Arzneimitteln.
Arzneimittel, die eine Verbindung der Formel 1 oder deren pharmazeutisch verträgliche Salze enthalten, können zum Beispiel oral, parenteral, intramuskulär, intravenös, rektal, nasal, durch Inhalation, subkutan oder durch eine geeignete transkutane Darreichungsform appliziert werden, wobei die bevorzugte Applikation von dem jeweiligen Erscheinungsbild der Erkrankung abhängig ist. Die Verbindungen der Formel 1 können dabei allein oder zusammen mit galenischen Hilfsstoffen zur Anwendung gemäss der Ansprüchen kommen, und zwar sowohl in der Veterinär- als auch in der Humanmedizin. Die Arzneimittel enthalten Wirkstoffe der Formel I und/oder deren pharmazeutisch verträgliche Salze im allgemeinen in einer Menge von 0.01 mg bis 1 g pro Dosiseinheit.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen, perkutanen oder intravenösen Applikation werden die verwendeten aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel bei einem etwa 75 kg schweren Patienten mindestens 0,001 mg/kg, vorzugsweise 0,1 mg/kg, bis höchstens 30 mg/kg, vorzugsweise 1 mg/kg Körpergewicht. In akuten Situationen, etwa unmittelbar nach Erleiden apnoetischer Zustände im Hochgebirge, können auch noch höhere Dosen notwendig werden. Insbesondere bei i.v.-Anwendung, etwa bei einem Infarktpatienten auf der Intensivstation können bis zu 300 mg/kg pro Tag notwendig werden. Die Tagesdosis kann auf eine oder mehrere, zum Beispiel bis zu 4 Einzeldosen verteilt werden.

### Versuchsbeschreibungen und Beispiele

Liste der verwendeten Abkürzungen:
- AMPA: durch α-Amino-3-hydroxy-5-methylisoxazol-4-propionat aktivierbare rezeptorgekoppelte Kanäle
- CA 1: CA = Cornu ammonis (Ammonshorn), CA Region 1 im Hippokampus
- EE: Ethylacetat
- EPSP: Exzitatorisches postsynaptisches Potential
- ES⁺: electron spray
- HEP: n-Heptan
- Konz. NH₃: gesättigte wäßrige NH₃-Lösung
- LTP: Langzeitpotenzierung
- LTP1: frühe LTP (Phase der LTP)
- MeOH: Methanol
- mp: Schmelzpunkt
- MS: Mass Spectroscopy
- NMDA: durch N-Methyl-D-Aspartat aktivierbare rezeptorgekoppelte Kanäle
- RT: Raumtemperatur
- STP: Short-term potentiation (Phase der LTP)
- THF: Tetrahydrofuran

### Beispiel 1: N-Diaminomethylen-4-(6,8-dichloro-2-methyl-1,2,3,4-tetrahydro-isochinolin-4-yl)-benzolsulfonamid, dihydrochlorid

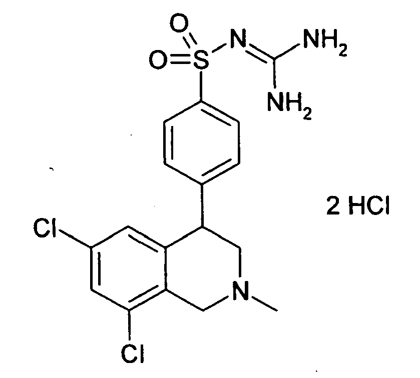

0.36g Guanidin werden unter Argon in 30 ml wasserfreiem THF suspendiert und 0.40g 4-(6,8-Dichloro-2-methyl-1,2,3,4-tetrahydro-isochinolin-4-yl)-benzolsulfonylchlorid (W02003048129) zugegeben. 24 h wurde bei RT gerührt, dann das THF abdestilliert. Der Rückstand wurde mit 10 ml Wasserr versetzt und der Niederschlag abfiltriert. Mit 10 ml Wasser wurde gewaschen und im Vakuum getrocknet. Der Feststoff wurde dann in 10 ml EE suspendiert und mit 10 ml einer gesättigten Lösung von HCl in Diethylether versetzt. Die flüchtigen Bestandteile wurden im Vakuum entfernt, der Rückstand in 10 ml EE suspendiert und 5 h bei RT gerührt. Der Niederschlag wurde dann abfiltriert und im Vakuum getrocknet. Und man erhielt 0.45 g, mp 140°C (Zersetzung).

| | |
|---|---|
| R_{f} (EE/HEP/CH₂Cl₂/MeOH/konz. NH₃ = 10:5:5:5:1) = 0.30 | MS (ES⁺) : 412 |

### Pharmakologische Daten:

### NHES-Testbeschreibung:

In diesem Test wurde die Erholung des intrazellulären pH-Wertes (pHi) von LAP1-Zellen, die unterschiedliche Subtypen des Natrium-Protonen-Austauschers (NHE) stabil exprimieren, nach einer Ansäuerung ermittelt. Diese Erholung setzt bei funktionsfähigem NHE auch unter bicarbonatfreien Bedingungen ein. Dazu wurde der pHᵢ mit dem pH-sensitiven Fluoreszenzfarbstoff BCECF (Molecular Probes, Eugene, OR, USA, eingesetzt wird die Vorstufe BCECF-AM) bestimmt. Die Zellen wurden zunächst mit BCECF (5 µM BCECF-AM) in NH₄Cl-Puffer (NH₄Cl-Puffer: 115 mM CholinCl, 20 mM NH₄Cl, 5 mM KCl 1 mM CaCl₂, 1 mM MgCl₂, 20 mM Hepes, 5 mM Glucose, mit 1 M KOH wird ein pH von 7,4 eingestellt) inkubiert. Die intrazelluläre Ansäuerung wurde durch Waschen der in NH₄Cl-Puffer inkubierten Zellen mit NH₄Cl freien Puffer (133,8 mM Cholinchlorid, 4,7 mM KCI, 1,25 mM Nach, 1,25 mM MgCl₂, 0,97 mM K₂HPO₄, 0,23 mM KH₂PO₄, 5 mM Hepes, 5 mM Glucose, mit 1 M KOH wird ein pH von 7,4 eingestellt) induziert. Nach dem Waschvorgang wurden 90 µl des NH₄Cl-freien Puffer auf den Zellen belassen. Die pH-Erholung wurde durch die Zugabe vom 90 µl Na⁺-haltigem Puffer (133,8 mM NaCl, 4,7 mM KCl, 1,25 mM CaCl₂, 1,25 mM MgCl₂, 0,97 mM Na₂HPO₄, 0,23 mM NaH₂PO₄, 10 mM Hepes, 5 mM Glucose, mit 1 M NaOH wird ein pH von 7,4 eingestellt) im Meßgerät (FLIPR, "Fluorometric Imaging Plate Reader", Molecular Devices, Sunnyvale, Ca., USA) gestartet. Die BCECF-Fluoreszenz wurde bei einer Anregungswellenlänge von 498 nm und dem FLIPR-Emissionsfilter 1 (Bandpass von 510 bis 570 nm) bestimmt. Die nachfolgenden Fluoreszenzänderungen als Maß der pH-Erholung wurden bei NHE5 für zwei Minuten registriert. Für die Berechnung der NHE-inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Na⁺-haltigem Puffer (s.o.), für die Bestimmung des 0%-Wertes in Na⁺-freien Puffer (s.o.) inkubiert. Die zu testenden Substanzen wurden in Na⁺-haltigem Puffer angesetzt. Die Erholung des intrazellulären pH's bei jeder getesteten Konzentration einer Substanz wurde in Prozent der maximalen Erholung ausgedrückt. Aus den Prozentwerten der pH-Erholung wurde mittels des Programms XLFit (idbs, Surrey, UK) der IC₅₀-Wert der jeweiligen Substanz für die einzelnen NHE-Subtypen berechnet.

| | NHE5 IC₅₀ [µM] |
|---|---|
| Beispiel 1 | 0.37 |

### Testbeschreibung: Langzeit-Experimente an Hippokampusschnitten (in vitro)

### Experimenteller Ansatz

Die LTP in der CA 1 Region ist die am besten charakterisierte LTP in vitro. Die Schichtung und Eingangsstruktur dieser Region erlaubt Feldpotentialmessungen über mehrere Stunden in vitro. In den NHE Studien wurde ein an den Thetarhythmus angelehnten schwachen Tetanus benutzt, der eine frühe LTP induziert, die innerhalb von drei Stunden auf den Ausgangswert zurückgeht (Journal of Neuroscience, 18(16), 6071(1998); Eur J Pharmacol. 502: 99-104, 2004). Es wurde kürzlich bestätigt, dass eine zunehmende Zahl von Thetafrequenzimpulsfolgen eine LTP von zunehmendem Ausmaß und Persistenz induziert (J Neurophysiol. 88:249-255, 2002), d.h. dass ein einzelner schwacher Stimulus nicht den maximal erzielbare gesättigten Typ der LTP, sondern eine ungesättigte LTP induziert. Sowohl Ausmaß (Behnisch, Reymann et al., Neurosci. Lett. 1998, 253(2): 91-94) als auch Persistenz (z.B. Neuropeptides 26: 421-427, 1994) diese LTP kann durch Substanzen verbessert oder verschlechtert werden. Die frühe LTP, die wir in unseren Untersuchungen erzeugen, ist ebenfalls ungesättigt. Man kann damit eine substanzinduzierte Verbesserung bzw. Verschlechterung der frühen LTP feststellen. Die untersuchte frühe LTP setzt sich aus der STP Komponente, die bekanntermaßen etwa 30 Minuten andauert (Nature 335: 820-824, 1988), und der LTP 1 Komponente, die gewöhnlich in den ersten 1 - 2 Stunden nach LTP Induktion (Learn Mem. 3: 1-24, 1996) andauert.
Die kurze (30-60 Minuten) Aufzeichnung der Ausgangswerte vor dem Tetanus erlaubt die Untersuchung von frühen Effekten der zu untersuchenden Substanz auf die normale, unstimulierte synaptische Übertragung. Da die hauptsächlichen exzitatorischen Synapsen glutamaterg sind (J Clin Neurophysiol. 9: 252-263, 1992), d.h. das monosynaptische Feld EPSP weitestgehend durch AMPA und nur zu einem wesentlich geringeren Anteil von NMDA Rezeptoren bestimmt wird, testet man damit indirekt gleichzeitig eine Wirkung auf die glutamaterge Transmission.

### Methode: Langzeit-Experimente an Hippokampusschnitten (in vitro)

Art der Tiere: .
Alter: 7-8 Wochen
Stamm: Wistar (Shoe Wist, Shoe)
Geschlecht: männlich
Zucht: Harlan Winkelmann GmbH, künstliches Licht (6-18 Uhr) und Tagesrhythmus

Präparation:
Betäubung: Schlag mit Eisenstab auf Genick
Tötung: Dekapitation

Hirnfreilegung: Öffnen der Schädeldecke durch Aufschneiden der Sagittalnaht des Schädels von dorsal nach ventral

Freilegung des Hippokampus: Das Hirn wurde zwischen den Hemisphären eingeschnitten und beginnend mit der rechten Hemisphäre der Hippokampus mit einem stumpfen Gegenstand herausgehoben.

Herstellung der Schnitte: Der freigelegte Hippokampus wurde auf einen Kühlblock mit feuchtem Filterpapier überführt und die überschüssige Feuchtigkeit mit Hilfe eines anderen Filterpapiers abgesaugt. Dieser so auf den Kühlblock befestigte Hippocampus wurde auf den Chopper gestellt und horizontal verdreht, bis der Hippokampus in einem entsprechenden Winkel zur Schneideklinge lag.

Schnittwinkel: Um die laminare Struktur des Hippokampus aufrecht zu erhalten war es notwendig, den Hippokampus in einen Winkel von etwa 70 Grad im Verhältnis zur Schnittklinge zu schneiden (Chopper).

Schnitt: In Abständen von 400 µm wurde der Hippokampus zerschnitten. Die Schnitte wurden mit Hilfe eines sehr weichen, stark benetzten Pinsels (Marderhaar) von der Klinge genommen und in ein Glasgefäß mit carbogenisierter gekühlter Nährlösung überführt. Die gesamte Präparationsdauer dauerte nicht länger als 5 min.

### Aufbewahrung der Schnitte:

Tauchschnitt: Die Schnitte lagen in einer temperierten Kammer (33° C) unter einem Flüssigkeitsspiegel von 1-3 mm. Die Durchflußrate betrug 2,5 ml/min. Die Vorbegasung erfolgte unter geringen Überdruck (etwa 1 atm) sowie über eine Mikrokanüle in der Vorkammer. Die Schnittkammer war mit der Vorkammer so verbunden, daß eine Minizirkulation aufrechterhalten werden konnte. Als Antrieb der Minizirkulation wurde das durch die Mikrokanüle ausströmende Carbogen eingesetzt.

Schnittadaptation: Die frisch präparierten Hippokampusschnitte wurden mindestens 1 h bei 33 °C in der Schnittkammer adaptiert.

### Bestimmung der Testreizstärke:

Reizstärke: fEPSP: 30% des EPSP-Maximums

### Messung der fokalen Potentiale

Stimulation: Mit Hilfe einer monopolaren Stimulationselektrode, die aus lackiertem Edelstahl bestand und eines stromkonstanten, biphasischen Reizgenerators (WPI A 365), wurden lokal die Schaffer-Kollateralen erregt (Spannung: 1-5 V, Impulsbreite einer Polarität 0.1 ms, Gesamtimpuls 0.2 ms).

Messung: Mit Hilfe von Glaselektroden (Borosilikatglas mit Filament, 1-5 MOhm, Durchmesser: 1.5 mm, Spitzendurchmesser: 3-20 µm), die mit normaler Nährlösung gefüllt waren, wurden aus dem Stratum radiatum die exzitatorischen postsynaptischen Potentiale (fEPSP) registriert. Die Messung der Feldpotentiale geschah gegenüber einer chlorierten Referenzelektrode aus Silber, die sich am Rande der Schnittkammer befand, mit Hilfe eines Gleichspannungsverstärker. Das Filtern der Feldpotentiale erfolgte über einen Low-Pass Filter (5 kHz).

Ermittlung der Feldpotentialwerte: Für die statistische Analyse der Experimente wurde der Anstieg (slope) der fEPSPs (fEPSP-Anstieg) ermittelt. Die Aufnahme, Analyse und Steuerung des Experimentes erfolgte mit Hilfe eines Softwareprogrammes (PWIN), welches in der Abteilung Neurophysiologie entwickelt wurde. Die Mittelwertbildung der fEPSP-Anstiegswerte zu den jeweiligen Zeitpunkten und die Konstruktion der Diagramme erfolgte mit Hilfe der Software EXCEL, wobei ein entsprechendes Makro die Aufnahme der Daten automatisierte.

### Nährmedium (Ringerlösung):

| Substanz | in mM | für 1 l in g |
|---|---|---|
| NaCl | 124 | 7.248 |
| KCl | 4.9 | 0.356 |
| MgSO4* 7H2O | 1.3 | 0.321 |
| CaCl2+ Wasser- frei | 2.5 | 0.368 |
| KH2PO4 | 1.2 | 0.164 |
| NaHCO3 | 25.6 | 2.152 |
| Glucose | 10 | 1.802 |
| Osmolarität in mOsm | 330 | |
| PH | 7.4 | |

Beispiel 1 wurde in DMSO gelöst und für die Experimente mit Ringerlösung auf die Endkonzentration verdünnt (Endkonzentration DMSO 0.01%).

### Versuchsablauf:

In den Kontrollexperimenten wurde zunächst für 60-120 Minuten die basale synaptische Transmission registriert. Anschließend wurden im Abstand von 200 ms viermal zwei Doppelpulsen mit einem Interpulsabstand der Doppelpulse von 10 ms und einer Breite der Einzelpulse von 0,2 ms (schwacher Tetanus) appliziert. Die resultierende Potenzierung der EPSPs wurde für mindestens 60 Minuten aufgezeichnet.

In den Experimenten zur Prüfung der Wirkung des NHE5-Inhibitors wurde ebenfalls zunächst für 60-120 Minuten die Basislinie aufgenommen. Das Einspülen des NHES-Inhibitors (10 µM) erfolgte 20 Minuten vor Stimulation. Es wurden wie in den Kontrollexperimenten viermal zwei Doppelpulse mit einem Interpulsabstand der Doppelpulse von 10 ms und einer Breite der Einzelpulse von 0,2,ms im Abstand von 200 ms appliziert. Die Substanz wurde 20 Minuten nach Stimulation ausgewaschen und die Potenzierung des EPSPs für mindestens 60 Minuten aufgezeichnet.

### Ergebnis:

Die Verbindung des Beispiels 1 hatte in der verwendeten Konzentration keine Eigenwirkung auf die synaptische Transmission.
Die Potenzierung nach Gabe des Beispiels 1 betrug 80 min nach Stimulus immer noch 137% der Basislinie, wohingegen die Potenzierung unter Kontrollbedingungen mit 113% der Basislinie schon fast wieder das Niveau der Basislinie erreicht hatte. Dies zeigt deutlich, dass bereits 10µM der Verbindung des Beispiels 1 die Aufrechterhaltung des schwachen LTP verbessern.

## Patentansprüche

1. Verwendung einer Verbindung der Formel I 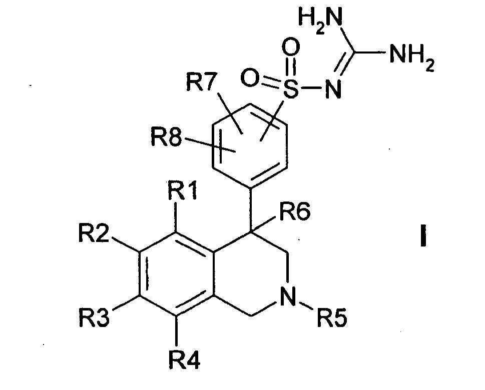 worin bedeuten:
R1, R2, R3 und R4
unabhängig voneinander Wasserstoff, F, Cl, Br, l, CN, NO₂ oder R11-(CₘH₂ₘ)-Aₙ-,
m Null, 1, 2, 3 oder 4;
n Null oder 1;
R11 Wasserstoff, Methyl oder CₚF₂ₚ₊₁;
A Sauerstoff, NH, N(CH₃) oder S(O)_{q};
p 1, 2 oder 3;
q Null, 1 oder 2;
R5 Wasserstoff, Alkyl mit 1, 2, 3, 4, 5 oder 6 C-Atomen oder Cycloalkyl mit 3, 4, 5 oder 6 C Atomen;
R6 Wasserstoff, OH, F, CF₃, Alkyl mit 1, 2, 3 oder 4 C-Atomen oder Cycloalkyl mit 3, 4, 5 oder 6 C Atomen;
R7 und R8 unabhängig voneinander Wasserstoff, F, Cl, Br, CN, CO₂R12, NR13R14 oder R16-(CₘₘH₂ₘₘ)-Eₙₙ-;
R12 Wasserstoff, Alkyl mit 1, 2, 3 oder 4 C-Atomen oder Cycloalkyl mit 3, 4, 5 oder 6 C-Atomen;
R13 und R14 unabhängig voneinander Wasserstoff, Alkyl mit 1, 2, 3 oder 4 C-Atomen oder Cycloalkyl mit 3, 4, 5 oder 6 C-Atomen;
oder
R13 und R14 bilden mit dem Stickstoffatom, an das sie gebunden sind, einen 4-, 5-, 6- oder 7-gliedrigen Ring, in dem eine CH₂-Gruppe durch NR15, S oder Sauerstoff ersetzt sein kann;
R15 Wasserstoff, Alkyl mit 1, 2, 3 oder 4 C-Atomen oder Cycloalkyl mit 3, 4, 5 oder 6 C-Atomen;
mm Null, 1, 2, 3 oder 4;
nn Null oder 1;
R16 Wasserstoff, Methyl oder CₚₚF₂ₚₚ₊₁;
E Sauerstoff oder S(O)_{qq};
pp 1, 2 oder 3;
qq Null, 1 oder 2;
sowie deren pharmazeutisch verträgliche Salze;
zur Herstellung eines Medikaments zur Behandlung von neurodegenerativen Erkrankungen, Gedächtnisstörungen und Demenzerkrankungen, sowie zur Gedächtnisverbesserung.

2. Verwendung nach Anspruch 1, wobei Verbindungen der Formel I verwendet werden, worin bedeuten
R1, R2, R3 und R4
unabhängig voneinander Wasserstoff, F, Cl, Br, CN oder R11-(CₘH₂ₘ)-Aₙ-;
m Null oder 1;
n Null oder 1;
R11 Wasserstoff, Methyl oder CₚF₂ₚ₊₁;
A Sauerstoff, NCH₃ oder S(O)_{q};
p 1 oder 2;
q Null, 1 oder 2;
R5 Wasserstoff, Methyl, Ethyl oder Cyclopropyl;
R6 Wasserstoff oder Methyl;
R7 und R8
unabhängig voneinander Wasserstoff, F, Cl, CN, CO₂R12, NR13R14 oder R16-(CₘₘH₂ₘₘ)-Eₙₙ-;
R12 Wasserstoff, Methyl oder Ethyl;
R13 und R14 unabhängig voneinander Wasserstoff, Alkyl mit 1, 2, 3 oder 4 C-Atomen oder Cycloalkyl mit 3, 4, 5 oder 6 C-Atomen;
oder
R13 und R14 bilden mit dem Stickstoffatom, an das sie gebunden sind, einen 5-, 6- oder 7-gliedrigen Ring, in dem eine CH₂-Gruppe durch NR15, S oder Sauerstoff ersetzt sein kann;
R15 Wasserstoff, Alkyl mit 1, 2, 3 oder 4 C-Atomen oder Cycloalkyl mit 3, 4, 5 oder 6 C-Atomen;
mm Null, 1 oder 2;
nn Null oder 1;
R16 Wasserstoff, Methyl oder CₚₚF₂ₚₚ₊₁;
E Sauerstoff oder S(O)_{qq};
pp 1 oder 2;
qq Null, 1 oder 2;
sowie deren pharmazeutisch verträglichen Salze.

3. Verwendung nach Anspruch 1 oder 2, wobei Verbindungen der Formel I verwendet werden, worin bedeuten
R1 und R3 Wasserstoff;
R2 und R4 unabhängig voneinander Wasserstoff, F, Cl, NH₂, NHCH₃ oder N(CH₃)₂;
R5 Wasserstoff, Methyl, Ethyl oder Cyclopropyl;
R6 Wasserstoff oder Methyl;
R7 und R8 Wasserstoff;
sowie deren pharmazeutisch verträglichen Salze.

4. Verwendung nach einem oder mehreren der Ansprüche 1 bis 3, wobei N-Diaminomethylen-4-(6,8-dichloro-2-methyl-1,2,3,4-tetrahydro-isochinolin-4-yl)-benzolsulfonamid oder ein pharmazeutisch verträgliches Salz davon verwendet wird.

5. Verwendung nach einem oder mehreren der Ansprüche 1 bis 4 zur Herstellung eines Medikaments zur Behandlung von Demenzerkrankungen.

6. Verwendung nach einem oder mehreren der Ansprüche 1 bis 5 zur Herstellung eines Medikaments zur Behandlung von Altersdemenz.

7. Verwendung nach einem oder mehreren der Ansprüche 1 bis 4 zur Herstellung eines Medikaments zur Behandlung von Alzheimer, vaskulären Demenzen, Kombinationen von Alzheimer und cereborvaskulären Erkrankungen, Tau-Mutationen, Prionen-Erkrankungen, Polyglutamin-Expansions-Erkrankungen und Parkinson.

8. Verwendung nach einem oder mehreren der Ansprüche 1 bis 4 zur Herstellung eines Medikaments zur Behandlung von Alzheimer, Multi-Infarkt-Demenz, Kombinationen von Alzheimer und cereborvaskulären Erkrankungen, Tau-Mutationen, Prionen-Erkrankungen, Chorea Huntington, spinocerebelläre Ataxien, und Parkinson.

9. Verwendung nach einem oder mehreren der Ansprüche 1 bis 4 zur Herstellung eines Medikaments zur Behandlung von sekundärer Demenzen nach und/oder bei Infektionen, Hirntraumen, Hirntumoren oder Intoxikationen.

10. Verwendung nach einem oder mehreren der Ansprüche 1 bis 4 zur Herstellung eines Medikaments zur Gedächtnisverbesserung.

## Claims

1. The use of a compound of the formula I 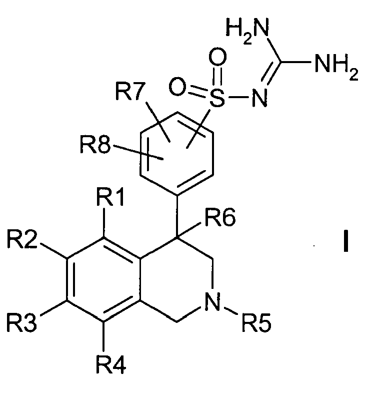 in which the meanings are:
R1, R2, R3 and R4 independently of one another hydrogen, F, Cl, Br, I, CN, NO₂ or R11-(CₘH₂ₘ)-Aₙ-;
m zero, 1, 2, 3 or 4;
n zero or 1;
R11 hydrogen, methyl or CₚF₂ₚ₊₁;
A oxygen, NH, N(CH₃) or S(O)_{q};
p 1, 2 or 3;
q zero, 1 or 2;
R5 hydrogen, alkyl having 1, 2, 3, 4, 5 or 6 C atoms or cycloalkyl having 3, 4, 5 or 6 C atoms;
R6 hydrogen, OH, F, CF₃, alkyl having 1, 2, 3 or 4 C atoms or cycloalkyl having 3, 4, 5 or 6 C atoms;
R7 and R8 independently of one another hydrogen, F, Cl, Br, CN, CO₂R12 , NR13R14 or R16- (CₘₘH₂ₘₘ) -Eₙₙ-;
R12 hydrogen, alkyl having 1, 2, 3 or 4 C atoms or cycloalkyl having 3, 4, 5 or 6 C atoms;
or
R13 and R14 independently of one another hydrogen, alkyl having 1, 2, 3 or 4 C atoms or cycloalkyl having 3, 4, 5 or 6 C atoms;
or
R13 and R14 form with the nitrogen atom to which they are bonded a 4, 5, 6 or 7 membered ring in which one CH₂ group may be replaced by NR15, S or oxygen;
R15 hydrogen, alkyl having 1, 2, 3 or 4 C atoms or cycloalkyl having 3, 4, 5 or 6 C atoms;
mm zero, 1, 2, 3 or 4;
nn zero or 1;
R16 hydrogen, methyl or CₚₚF_{2pp+1;}
E oxygen or S(O)_{qq};
pp 1, 2 or 3;
qq zero, 1 or 2;
and the pharmaceutically acceptable salts thereof; for producing a medicament for the treatment of neurodegenerative disorders, memory impairments and dementing disorders, and for improving memory.

2. The use as claimed in claim 1, where compounds of the formula I are used in which the meanings are
R1, R2, R3 and R4 independently of one another hydrogen, F, Cl, Br, CN or R11-(CₘH₂ₘ)-Aₙ-;
m zero or 1;
n zero or 1;
R11 hydrogen, methyl or CₚF₂ₚ₊₁;
A oxygen, NCH₃ or S(O)_{q};
p 1 or 2;
q zero, 1 or 2;
R5 hydrogen, methyl, ethyl or cyclopropyl;
R6 hydrogen or methyl;
R7 and R8 independently of one another hydrogen, F, Cl, CN, CO₂R12, NR13R14 or R16-(CₘₘH₂ₘₘ)-Eₙₙ-;
R12 hydrogen, methyl or ethyl;
R13 and R14 independently of one another hydrogen, alkyl having 1, 2, 3 or 4 C atoms or cycloalkyl having 3, 4, 5 or 6 C atoms;
or
R13 and R14 form with the nitrogen atom to which they are bonded a 5, 6 or 7 membered ring in which one CH₂ group may be replaced by NR15, S or oxygen;
R15 hydrogen, alkyl having 1, 2, 3 or 4 C atoms or cycloalkyl having 3, 4, 5 or 6 C atoms;
mm zero, 1 or 2;
nn zero or 1;
R16 hydrogen, methyl or CₚₚF₂ₚₚ₊₁;
E oxygen or S(O)_{qq};
pp 1 or 2;
qq zero, 1 or 2;
and the pharmaceutically acceptable salts thereof.

3. The use as claimed in claim 1 or 2, where compounds of the formula I are used in which the meanings are
R1 and R3 hydrogen;
R2 and R4 independently of one another hydrogen, F, Cl, NH₂, NHCH₃ or N(CH₃)₂;
R5 hydrogen, methyl, ethyl or cyclopropyl;
R6 hydrogen or methyl;
R7 and R8 hydrogen;
and the pharmaceutically acceptable salts thereof.

4. The use as claimed in one or more of claims 1 to 3, where N-diaminomethylene-4-(6,8-dichloro-2-methyl-1,2,3,4-tetrahydroisoquinolin-4-yl)benzenesulfonamide or a pharmaceutically acceptable salt thereof is used.

5. The use as claimed in one or more of claims 1 to 4 for producing a medicament for the treatment of dementing disorders.

6. The use as claimed in one or more of claims 1 to 5 for producing a medicament for the treatment of dementia in the elderly.

7. The use as claimed in one or more of claims 1 to 4 for producing a medicament for the treatment of Alzheimer's, vascular dementias, combinations of Alzheimer's and cerebrovascular disorders, tau mutations, prion disorders, polyglutamine expansion disorders and Parkinsonism.

8. The use as claimed in one or more of claims 1 to 4 for producing a medicament for the treatment of Alzheimer's, multi-infarct dementia, combinations of Alzheimer's and cerebrovascular disorders, tau mutations, prion disorders, Huntington's chorea, spinocerebellar ataxias, and Parkinsonism.

9. The use as claimed in one or more of claims 1 to 4 for producing a medicament for the treatment of secondary dementias following and/or associated with infections, brain traumas, brain tumors or intoxications.

10. The use as claimed in one or more of claims 1 to 4 for producing a medicament for improving memory.

## Revendications

1. Utilisation d'un composé de formule I 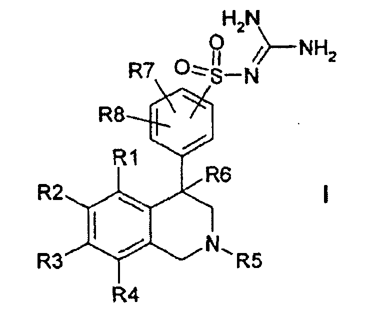 dans laquelle
R1, R2, R3 et R4 signifient, indépendamment l'un de l'autre, hydrogène, F, Cl, Br, I, CN, NO₂ ou R11- (CₘH₂ₘ) -Aₙ-;
m vaut zéro, 1, 2, 3 ou 4 ;
n vaut zéro ou 1 ;
R11 signifie hydrogène, méthyle ou CₚF₂ₚ₊₁ ;
A signifie oxygène, NH, N(CH₃) ou S (O)_{q} ;
p vaut 1, 2 ou 3 ;
q vaut zéro, 1 ou 2 ;
R5 signifie hydrogène, alkyle comprenant 1, 2, 3, 4, 5 ou 6 atomes de carbone ou cycloalkyle comprenant 3, 4, 5 ou 6 atomes de carbone ;
R6 signifie hydrogène, OH, F, CF₃, alkyle comprenant 1, 2, 3 ou 4 atomes de carbone ou cycloalkyle comprenant 3, 4, 5 ou 6 atomes de carbone ;
R7 et R8 signifient, indépendamment l'un de l'autre, hydrogène, F, Cl, Br, CN, CO₂R12 ou NR13R14 ou R16- (CₘₘH₂ₘₘ) -Eₙₙ- ;
R12 signifie hydrogène, alkyle comprenant 1, 2, 3 ou 4 atomes de carbone ou cycloalkyle comprenant 3, 4, 5 ou 6 atomes de carbone ;
R13 et R14 signifient, indépendamment l'un de l'autre, hydrogène, alkyle comprenant 1, 2, 3 ou 4 atomes de carbone ou cycloalkyle comprenant 3, 4, 5 ou 6 atomes de carbone ;
ou
R13 et R14 forment avec l'atome d'azote auquel ils sont liés, un cycle de 4, 5, 6 ou 7 chaînons, dans lequel un groupe CH₂ peut être remplacé par NR15, S ou oxygène ;
R15 signifie hydrogène, alkyle comprenant 1, 2, 3 ou 4 atomes de carbone ou cycloalkyle comprenant 3, 4, 5 ou 6 atomes de carbone ;
mm vaut zéro, 1, 2, 3 ou 4 ;
nn vaut zéro ou 1 ;
R16 signifie hydrogène, méthyle ou CₚₚF₂ₚₚ₊₁ ;
E signifie oxygène ou S(O)qq ;
pp vaut 1, 2 ou 3 ;
qq vaut zéro, 1 ou 2 ;
ainsi que leurs sels pharmaceutiquement acceptables ; pour la préparation d'un médicament destiné au traitement de maladies de neurodégénérescence, de troubles de la mémoire et de maladies de démence, ainsi qu'à l'amélioration de la mémoire.

2. Utilisation selon la revendication 1, où des composés de formule I sont utilisés, dans laquelle :
R1, R2, R3 et R4 signifient, indépendamment l'un de l'autre, hydrogène, F, Cl, Br, CN, R11-(CₘH₂ₘ)-Aₙ- ;
m vaut zéro ou 1 ;
n vaut zéro ou 1 ;
R11 signifie hydrogène, méthyle ou CₚF_{2p+1;}
A signifie oxygène, NCH₃ ou S(O)_{q} ;
p vaut 1 ou 2 ;
q vaut zéro, 1 ou 2 ;
R5 signifie hydrogène, méthyle, éthyle ou cyclopropyle ;
R6 signifie hydrogène ou méthyle ;
R7 et R8 signifient, indépendamment l'un de l'autre, hydrogène, F, Cl, CN, CO₂R12 ou NR13R14 ou R16-(CₘₘH₂ₘₘ) -Eₙₙ- ;
R12 signifie hydrogène, méthyle ou éthyle ;
R13 et R14 signifient, indépendamment l'un de l'autre, hydrogène, alkyle comprenant 1, 2, 3 ou 4 atomes de carbone ou cycloalkyle comprenant 3, 4, 5 ou 6 atomes de carbone ;
ou
R13 et R14 forment avec l'atome d'azote auquel ils sont liés, un cycle de 5, 6, ou 7 chaînons, dans lequel un groupe CH₂ peut être remplacé par NR15, S ou oxygène ;
R15 signifie hydrogène, alkyle comprenant 1, 2, 3 ou 4 atomes de carbone ou cycloalkyle comprenant 3, 4, 5 ou 6 atomes de carbone ;
mm vaut zéro, 1 ou 2 ;
nn vaut zéro ou 1 ;
R16 signifie hydrogène, méthyle ou CₚₚF₂ₚₚ₊₁ ;
E signifie oxygène ou S(O)_{qq} ;
pp vaut 1 ou 2 ;
qq vaut zéro, 1 ou 2 ;
ainsi que leurs sels pharmaceutiquement acceptables.

3. Utilisation selon la revendication 1 ou 2, où des composés de formule I sont utilisés, dans laquelle :
R1 et R3 signifient hydrogène ;
R2 et R4 signifient, indépendamment l'un de l'autre, hydrogène, F, Cl , NH₂, NHCH₃ ou N (CH₃)₂ ;
R5 signifie hydrogène, méthyle, éthyle ou cyclopropyle ;
R6 signifie hydrogène ou méthyle ;
R7 et R8 signifient hydrogène ;
ainsi que leurs sels pharmaceutiquement acceptables.

4. Utilisation selon l'une ou plusieurs des revendications 1 à 3, où on utilise du N-diaminométhylène-4-(6,8-dichloro-2-méthyl-1,2,3,4-tétrahydroisoquinoléin-4-yl)-benzènesulfonamide ou un sel pharmaceutiquement acceptable de celui-ci.

5. Utilisation selon l'une ou plusieurs des revendications 1 à 4 pour la préparation d'un médicament destiné au traitement de maladies de démence.

6. Utilisation selon l'une ou plusieurs des revendications 1 à 5 pour la préparation d'un médicament destiné au traitement de la démence sénile.

7. Utilisation selon l'une ou plusieurs des revendications 1 à 4 pour la préparation d'un médicament destiné au traitement de la maladie d'Alzheimer, de démences vasculaires, de combinaisons de maladies d'Alzheimer et cérébrovasculaires, de mutations de Tau, de maladies de prion, de maladies d'expansion de la polyglutamine et de la maladie de Parkinson.

8. Utilisation selon l'une ou plusieurs des revendications 1 à 4 pour la préparation d'un médicament destiné au traitement de la maladie d'Alzheimer, de la démence d'un multi-infarctus, de combinaisons de maladies d'Alzheimer et cérébrovasculaires, de mutations de Tau, de maladies de prion, de la chorée de Huntington, d'ataxies spino-cérébellaires et de la maladie de Parkinson.

9. Utilisation selon l'une ou plusieurs des revendications 1 à 4 pour la préparation d'un médicament destiné au traitement de démences secondaires après et/ou lors d'infections, de traumas du cerveau, de tumeurs du cerveau ou d'intoxications.

10. Utilisation selon l'une ou plusieurs des revendications 1 à 4 pour la préparation d'un médicament destiné à l'amélioration de la mémoire.
